# EUROPEAN PATENT SPECIFICATION

(11) **EP 1 601 392 B1**
(45) Date of publication and mention of the grant of the patent: **29.04.2009**
(21) Application number: 04718550.9
(22) Date of filing: 08.03.2004
(51) Int. Cl.: A61L 31/18, A61P 9/00

(54) **COMPOSITIONS FOR USE IN EMBOLIZING BLOOD VESSELS COMPRISING HIGH LEVELS OF CONTRAST AGENT**
ZUSAMMENSETZUNGEN ENTHALTEND HOHE KONZENTRATION VON KONTRASTMITTEL ZUR EMBOLISIERUNG VON BLUTGEFASSEN
COMPOSITIONS UTILISEES POUR EMBOLISER DES VAISSEAUX SANGUINS, PRESENTANT DE FORTES CONCENTRATIONS EN AGENT DE CONTRASTE

(30) Priority: 07.03.2003 US 452555 P
(43) Date of publication of application: 07.12.2005
(73) Proprietor: Micro Therapeutics, Inc., Irvine CA 92618 (US)
(72) Inventor: BEIN, Richard, S., San Clemente, CA 92673 (US); GREFF, Richard, J., St. Pete Beach, FL 33706 (US); STRAUSS, Brian M., Trabuco Canyon, CA 92679 (US); CANFIELD, Brian, San Clemente, CA 92673 (US)
(74) Representative: Wibbelmann, Jobst
(86) International application number: PCT/US2004/007017
(87) International publication number: WO 2004/080503

(56) References cited:
- WO-A-00/56370
- WO-A-00/71170
- US-A- 5 667 767
- US-A- 5 695 480
- US-A- 5 925 683
- US-A1- 2002 187 102
- US-B1- 6 454 738
- MOTTU F ET AL: "Iodine-containing cellulose mixed esters as radiopaque polymers for direct embolization of cerebral aneurysms and arteriovenous malformations." January 2002 (2002-01), BIOMATERIALS. JAN 2002, VOL. 23, NR. 1, PAGE(S) 121 - 131 , XP004322627 ISSN: 0142-9612 page 122, paragraph 2; tables 1,2
- WRIGHT K C ET AL: "Experimental evaluation of cellulose acetate NF and ethylene-vinyl alcohol copolymer for selective arterial embolization." JOURNAL OF VASCULAR AND INTERVENTIONAL RADIOLOGY : JVIR. OCT 1999, vol. 10, no. 9, October 1999 (1999-10), pages 1207-1218, XP000800903 ISSN: 1051-0443

## Description

### Cross Reference to Related Applications

This application claims the benefit under 35 USC § 119(e) to U.S. Provisional Application Serial No. 60/452,555, filed March 7, 2003 which is hereby incorporated by reference in its entirety.

### BACKGROUND OF THE INVENTION

### Field of the Invention

This invention is directed to novel compositions suitable for use in embolizing blood vessels. In particular, this invention is directed to embolizing compositions comprising a biocompatible polymer, a biocompatible solvent, and a high concentration of contrast agent.

### References

The following references are cited in this application as superscript numbers:
¹ Greff, et al., U.S. Patent No. 5,695,480, *Novel Embolizing Compositions,* issued December 9, 1997.
² Greff, et al., U.S. Patent No. 5,667,767, *Compositions for Use in Embolizing Blood Vessels,* issued September 16, 1997.
³ Whalen, et al., allowed U.S. Patent No. 6,531,111, *Novel High Viscosity Embolizing Compositions,* issued March 11, 2003.
⁴ Whalen, et art, U.S. Publication No. 2003-0223955, *Methods for Embolizing Aneurysmal Sites With a High Viscosity Embolizing Composition,* published December 4, 2003.

All of the above references are herein incorporated by reference in their entirety to the same extent as if each individual reference was specifically and individually indicated to be incorporated herein by reference in its entirety.

### State of the Art

Embolization of blood vessels is conducted for a variety of reasons, including for treatment of tumors, lesions (such as aneurysms), arteriovenous malformations ("arms"), arteriovenous fistula ("AVF"), uncontrolled bleeding, and the like.

Embolization of blood vessels is preferably accomplished via catheter techniques which permit the selective placement of the catheter at the vascular site to be embolized. Recent advances in catheter technology and in angiography now permit endovascular intervention, including the treatment of what would otherwise have been inoperable lesions. Specifically, the development of microcatheters and guide wires capable of providing access to vessels as small as 1 mm in diameter allows for endovascular treatment of many lesions.

Endovascular treatment regimens preferably include the use of embolizing compositions containing a water-insoluble, radiopaque contrast agent. The radiopaque contrast agent allows the treating physician to visualize delivery of the embolic composition to the vascular site via conventional techniques such as fluoroscopy. The use of a water-insoluble contrast agent is also beneficial for post-treatment procedures. For example, the presence of the contrast agent allows the treating physician to visualize the embolized mass during surgery or retreatment and to monitor the disease condition.

Visualization is particularly necessary when performing embolization using catheter delivery techniques. The ability to visualize the composition helps ensure not only that the composition is being delivered to the intended vascular site, but also that it is being delivered in the correct amount. The latter advantage is particularly helpful in the treatment of aneurysms, where the aneurysmal sac is intended to be filled but the adjoining blood vessel is not. Accordingly, in such treatments, the amount of embolic composition delivered is selected to substantially fill but not overflow the aneurysmal sac. If less than this amount of embolic composition is delivered to the aneurysmal sac, the patient will be left with an active aneurysm. In some cases this can be more dangerous than the untreated aneurysm. If the amount of embolic composition delivered is greater than what is required to fill the aneurysm, the composition will overflow into the adjoining blood vessel. The excess composition can then embolize the adjoining blood vessel. In the case where the affected blood vessel is in or leads to a critical body organ, such as the brain, permanent damage due to cessation in blood flow will result.

In the treatment of AVMs, low viscosity embolic compositions can be used to facilitate delivery deep into the vascular bed. The use of such embolic compositions containing high concentrations of water-insoluble contrast agents permits the attending clinician to more effectively visualize and control the composition while penetrating the vascular bed of the treated AVM.

Embolic compositions for catheter delivery preferably comprise a biocompatible solvent, a biocompatible polymer, and a water-insoluble contrast agent suspended therein. The biocompatible solvent is miscible or soluble in blood or other body fluid and also solubilizes the biocompatible polymer during delivery. The biocompatible polymer is selected to be soluble in the biocompatible solvent, but insoluble in blood or other body fluid. The water-insoluble contrast agent is suspended in the composition and, as above, permits the physician to fluoroscopically visualize catheter delivery of the composition. Upon contact with the blood or other body fluid, the biocompatible solvent dissipates from the embolic composition, whereupon the biocompatible polymer precipitates in the presence of the water-insoluble contrast agent and embolizes the blood vessel.

Over time, the art became aware of complications in this procedure. Such complications include inconsistent visibility of the embolic composition during catheter delivery of the composition. Inconsistent visibility can result in either the under-filling or over-filling of the vascular site to be embolized, thereby producing an unsatisfactory result.

Heretofore, the art recognized that the average particle size of the water-insoluble contrast agent was critical to providing embolic compositions which can be consistently visualized during catheter delivery. Specifically, it was found that fluoroscopic visualization of the embolic composition was enhanced by employing a water-insoluble contrast agent with an average particle size of about 10 µm or less. Notwithstanding the benefits achieved by the use of such compositions, visibility of the embolic composition under fluoroscopy remains a major concern, particularly in those endovascular surgical situations requiring the use of smaller amounts of composition.

With regard to the above, the art has disclosed the use of up to 40 weight percent of the contrast agent into the embolic composition.^{1,2} However, the mere addition of additional contrast agent into the embolic composition in order to enhance fluoroscopic visibility poses several practical concerns.

One practical concern is that of ensuring the embolic composition is suited for microcatheter injection, as embolic compositions are typically delivered through microcatheters. Accordingly, the quantity of water-insoluble biocompatible contrast agent suspended in the composition must result in a composition with adequate flowability through the microcatheter. That is to say that the contrast agent cannot plug the microcatheter or cause high injection pressures.

Another practical concern is the level of embolization precision achieved with the embolic composition. The use of higher quantities of water-insoluble biocompatible contrast agent must result in a coherent precipitate formed *in vivo* which minimizes fragmentation and possible embolization of unintended vascular sites. It is believed that the cohesive precipitate formed is a matrix of water-insoluble contrast agent encapsulated within the water-insoluble biocompatible polymer. Accordingly, higher amounts of water-insoluble contrast agent may not result in a cohesive precipitate particularly at low concentrations of polymer as found in embolic compositions having low viscosities, *i.e*., less than about 100 cSt at 40 C.

Yet another practical concern is that embolic compositions are delivered parenterally, including intravascular delivery. Due to the parenteral nature of delivery, the patient's filtering mechanisms, utilized with enteral delivery, are not available. As such, the embolic compositions as injected must not pose a danger to the patient. The use of higher quantities of water-insoluble biocompatible contrast agent must result in a composition whose population of large particles is small enough not to pose a health threat

In view of the above, embolic compositions capable of penetrating into small vessels and providing greater visibility under fluoroscopy would be particularly beneficial.

### SUMMARY OF THE INVENTION

This invention is directed to the novel and unexpected discovery that fluidic embolic compositions with specified higher concentrations of water-insoluble biocompatible contrast agent suspended therein retain efficacy for use in endovascular surgical procedures while offering safe, greater fluoroscopic visualization, provided that the composition employs ae. 45- 60 weight percent water-insoluble contrast agent and has a ratio of biocompatible polymer to the water-insoluble biocompatible contrast agent of 0.055 or greater. When so employed, it has unexpectedly been found that a cohesive precipitate is formed which inhibits the undesired shedding of particles *in vivo*.

In a preferred embodiment, the resulting precipitate formed from the embolic composition has a releasable number of particles of water-insoluble biocompatible contraste agent equal to or greater than 10 microns of 25 particles or less per milliliter of solution used to evaluate the number of such particles.

In another preferred embodiment, the resulting precipitate formed from the embolic composition has a releasable number of particles of water-insoluble biocompatible contrast agent equal to or greater than 25 microns of 3 particles or less per milliliter of solution used to evaluate the number of such particles.

Without being limited to any theory, it is believed that the maximum concentration of the water-insoluble biocompatible contrast agent ensures the flowability of the embolic composition through the microcatheter while not inhibiting injection or plugging the microcatheter. In addition, the ratio of biocompatible polymer to the water-insoluble contrast agent ensures that a cohesive precipitate forms *in vivo*. This latter property is particularly relevant in low viscosity embolic compositions employing reduced amounts of biocompatible polymer and enhanced amounts of water-insoluble biocompatible contrast agent.

Accordingly, in one of its composition aspects, this invention is directed to a composition comprising:
a) a biocompatible polymer,
b) a biocompatible solvent; and
c) from greater than 45 to 60 weight percent of a water-insoluble, biocompatible contrast agent;
wherein the ratio of biocompatible polymer to the water-insoluble biocompatible contrast agent is 0.055 or greater; and
further wherein the weight percent of each component is based on the total weight of the composition.

Preferably, the water-insoluble biocompatible contrast agent is employed at a concentration of from greater than 45 to 55 weight percent, and, even more preferably, from greater than 45 to 50 weight percent, based on the total weight of the composition.

In a preferred embodiment, the average particle size of the water-insoluble biocompatible contrast agent should be less than 5 microns, and more preferably from 2 microns to 3 microns.

Preferably, the water-insoluble, biocompatible contrast agent is selected from the group consisting of barium sulfate, tantalum, tantalum oxide, gold, platinum and tungsten.

Preferably, the biocompatible polymer is employed at a concentration of from 2 to 40 weight percent, more preferably, from 2 to 30 weight percent and, even more preferably, from 2 to 20 weight percent, based on the total weight of the composition.

Preferably, the biocompatible polymer is selected from the group consisting of cellulose acetates, ethylene vinyl alcohol copolymers, hydrogels, polyacrylonitrile, polyvinylacetate, cellulose acetate butyrate, nitrocellulose, copolymers of urethane/catbonate, copolymers of styrene/maleic acid, and mixtures thereof

Preferably, the concentration of biocompatible solvent is from 20 weight percent to less than 58 weight percent. More preferably, the biocompatible solvent is employed in an amount of from 20 to 57 weight percent, and even more preferably, from 40 to 55 weight percent, based on the total weight of the composition.

Preferably, the biocompatible solvent is selected from the group consisting of dimethylsulfoxide ("DMSO"), ethanol, ethyl lactate and acetone.

In one preferred embodiment, the resulting precipitate formed from the embolic composition has a releasable number of particles of water-insoluble biocompatible contrast agent equal to or greater than 10 microns of 25 particles or less per milliliter of solution used to evaluate the number of such particles.

In another preferred embodiment, the resulting precipitate formed from the embolic composition has a releasable number of particles of water-insoluble biocompatible contrast agent equal to or greater than 25 microns of about 3 particles or less per milliliter of solution used to evaluate the number of such particles.

In one of its kit aspects, this invention is directed to a kit of parts comprising :
a) an embolic composition which comprises
   i) a biocompatible polymer ;
   ii) a biocompatible solvent; and
   iii) from 45 to 60 weight percent of a water- insoluble, biocompatible contrast agent;
   wherein the ratio of biocompatible polymer to the water-insoluble biocompatible contrast agent is 0.055 or greater,
   and further wherein the weight percent of each component is based on the total weight of the composition; and
b) a catheter.

In a preferred embodiment, the kit further comprises a microballoon catheter to attenuate or arrest blood flow.

### BRIEF DESCRIPTION OF THE DRAWING

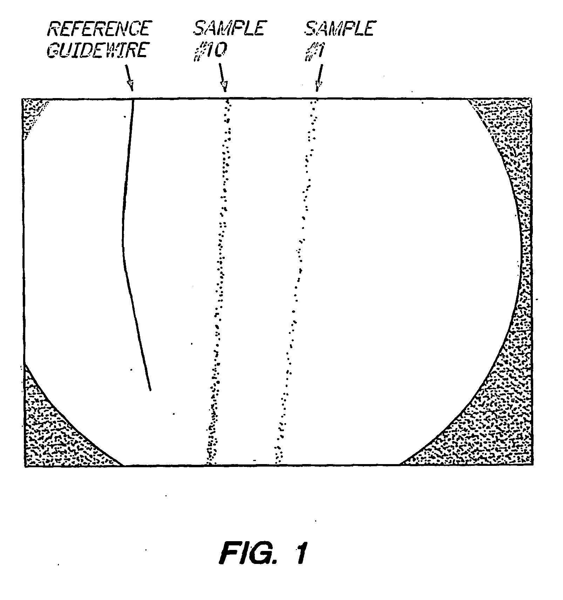

Figure 1 illustrates the radiopacity of a conventional embolic composition ("Sample #1") and of a composition comprising a higher level of biocompatible contrast agent ("Sample #10"), in comparison to a reference guidewire (a Micro Therapeutics SilverSpeed .010" diameter guidewire).

### DETAILED DESCRIPTION OF THE INVENTION

This invention is directed, in part, to novel compositions for embolizing blood vessels which are particularly well-suited for treating vascular lesions via catheter delivery of the composition. The claimed compositions are particularly well-suited for treatment of vascular lesions because they flow well in a microcatheter, the cohesive precipitate formed minimizes fragmentation and possible embolization of unintended sites, and because the compositions are especially safe for administration.

Prior to discussing this invention in further detail, the following terms will first be defined:

The term "biocompatible polymer" refers to polymers which, in the amounts employed, are non-toxic, chemically inert, substantially non-immunogenic, when used internally in a mammalian patient are soluble in the biocompatible solvent and which are substantially insoluble in blood. Suitable biocompatible polymers include, by way of example, cellulose acetates (including cellulose diacetate), ethylene vinyl alcohol copolymers, hydrogels (*e.g*., acrylics), polyacrylonitrile, polyvinylacetate, cellulose acetate butyrate, nitrocellulose, copolymers of urethane/carbonate, copolymers of styrene/maleic acid, and mixtures thereof. Preferably, the biocompatible polymer is also substantially non-inflammatory when employed *in vivo.*

The particular biocompatible polymer employed is not critical and is selected relative to the viscosity of the resulting polymer solution, the solubility of the biocompatible polymer in the biocompatible solvent, and the like. Such factors are well within the skill of the art.

The compositions described herein preferably have a viscosity of at least 15 cSt at 40°C and more preferably of from about 15 to 20,000 cSt at 40°C.

Lower viscosity compositions such as those having a viscosity of less than 150 cSt at 40°C can be achieved by the use of lower concentrations of biocompatible polymer, the use of a lower molecular weight polymer, or combinations thereof. The preparation of lower viscosity embolic compositions is well within the skill of the art. Such lower viscosity compositions are of particular utility in treating AVMs, tumors, and the like.

Higher viscosity compositions such as those having a viscosity of 150 cSt or higher at 40°C can be achieved by the use of higher concentrations of biocompatible polymer, the use of a higher molecular weight polymer, or combinations thereof The preparation of higher viscosity embolic compositions is well within the skill of the art. Such high-viscosity compositions are described by Whalen, et al.^{3,4} Higher viscosity compositions are of particular utility in treating aneurysms and other related arterial diseases.

Accordingly, adjustment of the viscosity of the composition can be readily achieved by mere adjustment of the molecular weight of the polymer composition and/or by increasing the concentration of the polymer in the composition.

Preferred biocompatible polymers include ethylene vinyl alcohol (EVOH) and cellulose diacetate. Ethylene vinyl alcohol copolymers comprise residues of both ethylene and vinyl alcohol monomers. Small amounts (*e.g*., less than 5 mole percent) of additional monomers can be included in the polymer structure or grafted thereon provided such additional monomers do not alter the embolizing properties of the composition. Such additional monomers include, by way of example only, maleic anhydride, styrene, propylene, acrylic acid, vinyl acetate and the like.

Biocompatible polymers are either commercially-available or can be prepared by art-recognized procedures. For example, polymers are typically prepared by conventional techniques such as radical, thermal, UV, gamma-irradiation, or electron beam-induced polymerization employing, as necessary, a polymerization catalyst or polymerization initiator to provide for the polymer composition. The specific manner of polymerization is not critical and the polymerization techniques employed do not form a part of this invention.

In order to maintain solubility in the biocompatible solvent, the polymers described herein are preferably not cross-linked.

The term "biocompatible solvent" refers to an organic material liquid at least at body temperature of the mammal in which the biocompatible polymer is soluble and, in the amounts used, is substantially non-toxic. Suitable biocompatible solvents include, by way of example, dimethylsulfoxide, analogues/homologues of dimethylsulfoxide, ethanol, ethyl lactate, acetone, and the like. Aqueous mixtures with the biocompatible solvent can also be employed provided that the amount of water employed is sufficiently small that the dissolved polymer precipitates upon contact with the blood. Preferably, the biocompatible solvent is dimethylsulfoxide.

The term "embolizing" refers to a process wherein a material is injected into a blood vessel/vascular site which, in the case of, for example, aneurysms, fills or plugs the aneurysmal sac and/or encourages clot formation so that blood flow into the aneurysm ceases, and in the case of AVMs and AVFs, forms a plug or clot to control/reroute blood flow to permit proper tissue perfusion. Embolization of the blood vessel is, therefore, important in preventing/controlling bleeding due to lesions (*e.g*., organ bleeding, gastrointestinal bleeding, vascular bleeding as well as bleeding associated with an aneurysm). In addition, embolization can be used to ablate diseased tissue (*e.g*., tumors, etc.) by cutting off its blood supply.

The term "encapsulation" as used relative to the contrast agent being encapsulated in the polymer precipitate is not meant to infer any physical entrapment of the contrast agent within the precipitate, much as a capsule encapsulates a medicament. Rather, this term is used to mean that an integral, coherent precipitate forms which does not separate into individual components.

The term "water-insoluble biocompatible contrast agent" refers to a biocompatible, water-insoluble (*i.e*., has a water solubility of less than 0.01 mg/ml at 20°C), radiopaque material capable of being monitored during injection into a mammalian subject by, for example, radiography. Examples of biocompatible water-insoluble contrast agents include tantalum, tantalum oxide, and barium sulfate, which are commercially available in the proper form for *in vivo* use. Methods for preparing such water-insoluble biocompatible contrast agents having an average particle size of 5 µm or less are described below. Other water-insoluble contrast agents include gold, tungsten, and platinum.

The term "releasable" as used in conjunction with the phrase releasable number of particles of water-insoluble biocompatible contrast agent per milliliter of solution" refers to the number of particles released from the precipitate formed from the polymer/water-insoluble contrast agent as described herein when tested in the manner of Example 3 hereof

### Compositions

The polymer compositions employed herein are prepared by conventional methods, whereby each of the components is added and the resulting composition mixed together until the overall composition is substantially homogeneous.

For example, polymer compositions can be prepared by adding sufficient amounts of the biocompatible polymer to the biocompatible solvent to achieve the effective concentration for the polymer composition. Preferably, the polymer composition will comprise from 2 to 40 weight percent of the biocompatible polymer composition based on the total weight of the polymer composition, more preferably from 2 to 30 weight percent and even more preferably from 2 to 20 weight percent. If necessary, gentle heating and stirring can be used to effect dissolution of the biocompatible polymer into the biocompatible solvent, *e.g*., 1-2 hours at 55°C.

Sufficient amounts of the contrast agent are then added to the biocompatible solvent to achieve the effective concentration for the complete composition. Preferably, the composition will comprise from greater than 45 to 60 weight percent of the contrast agent, more preferably, from greater than 45 to 55, and even more preferably from 45 to 50 weight percent, based on the total weight of the composition. Insofar as the contrast agent is not soluble in the biocompatible solvent, stirring is employed to effect homogeneity of the resulting suspension.

As to the biocompatible polymer and water-insoluble contrast agent, the amounts of each component are selected such that the ratio of biocompatible polymer to the water-insoluble biocompatible contrast agent is 0.055:1 or greater and, preferably from 0.07:1 to 0.90:1.

Preferably, the average particle size of the contrast agent be maintained at 5 µm or less. It is more preferred that the average particle size of the contrast agent range from 2 µm to 3 µm

In one preferred embodiment, the appropriate particle size of the contrast agent is prepared, for example, by fractionation. In such an embodiment, a water-insoluble contrast agent, such as tantalum, having an average particle size of less than 20 µm is added to an organic liquid such as ethanol (absolute), preferably in a clean environment Agitation of the resulting suspension followed by settling for approximately 40 seconds permits the larger particles to settle faster. Removal of the upper portion of the organic liquid followed by separation of the liquid from the particles results in a reduction of the particle size which is confirmed under an optical microscope. The process is optionally repeated until both a desired average particle size is reached and the maximum number of particles exceeding a given size is also reached.

The particular order of addition of components to the biocompatible solvent is not critical and stirring of the resulting suspension is conducted as necessary to achieve substantial homogeneity of the composition. Preferably, mixing/stirring of the composition is conducted under an anhydrous atmosphere at ambient pressures. The resulting composition is optionally heat-sterilized and then stored, preferably in sealed amber bottles or vials until needed.

### Methods

The compositions described above can then be employed in methods for the catheter-assisted Embolization of mammalian blood vessels. In Such methods, a sufficient amount of this composition is introduced into the selected blood vessel via a catheter delivery means under fluoroscopy so that upon precipitation of the polymer, the blood vessel is embolized. The particular amount of embolizing composition employed is dictated by the total volume of the vasculature to be embolized, the concentration of polymer in the composition, the rate of precipitation (solids formation) of the polymer, etc. Such factors are well within the skill of the art.

One particularly preferred method for catheter delivery of the embolizing compositions of this invention to the selected vascular site is via a small diameter medical catheter. The particular catheter employed is not critical, provided that catheter components are compatible with the embolizing composition (*i.e.,* the catheter components will not readily degrade in the embolizing composition). In this regard, it is preferred to use polyethylene in the catheter components because of its inertness in the presence of the embolizing composition described herein. Other materials compatible with the embolizing compositions can be readily determined by the skilled artisan and include, for example, other polyolefins, fluoropolymers (*e.g*., Teflon^{™}), silicone, etc.

When delivered by catheter, the injection rate dictates, in part, the form of the precipitate at the vascular site. Specifically, low injection rates of approximately 0.05 to 0.1 cc/minute will provide for a precipitate in the form of a kernel or nodule which is particularly beneficial for site specific embolization because the precipitate forms primarily at the point of injection. Also, when dimethylsulfoxide ("DMSO") is used to prime the catheter, too rapid of an injection of DMSO into the vascular site can cause vascular spasms and, accordingly, should be avoided and/or the use of anti-spasmodic drugs such as papaverine can be employed if spasms arise.

One particularly preferred method for the catheter injection of the composition of this invention into an aneurysmal site is as follows:
1. Place distal tip of the delivery catheter within the aneurysmal site, preferably about 2/3 into the sac fundus.
2. Flush delivery catheter with saline (*e.g*., about 5 cc).
3. Fill dead space of delivery catheter with DMSO (*e.g*., 0.25 cc).
4. Inject a desired amount of embolizing composition (*e.g*, 0.20 cc) into the delivery catheter channel at a rate of less than 0.1 cc/minute.
5. Stop injection and wait until the DMSO has been sufficiently flushed from the site (*e.g*., 1 minute).
6. Slowly inject the embolizing composition until the sac of the aneurysm is filled as visualized by fluoroscopy. A separate liquid contrast agent can be injected, for example, through a separate catheter proximate to the aneurysm, during the procedure as needed to determine percent of aneurysm fill.
7. The delivery catheter is detached:
   7.1 Wait a sufficient amount of time (*e.g*., 10 minutes) to permit solidification of the embolizing composition.
   7.2 Aspirate the syringe (*e.g*., 0.20 cc).
   7.3 Remove slack from the delivery catheter.
   7.4 Detach with quick pull.

In an alternative embodiment of the invention, the above method may be modified as follows, step 6 may comprise some or all of the following sub-steps:
6. Embolize the aneurysm as follows:
   6.1 Slowly inject the embolizing composition until a nidus forms in the sac of the aneurysm.
   6.2 Wait for a period sufficient to allow perfusion of the site (*e.g*., 1-3 minutes) and thereby removal of the biocompatible solvent; this promotes solidification of the precipitate.
   6.3 Slowly inject additional embolizing composition to grow the forming precipitate in the sac of the aneurysm.
   6.4 Repeat steps 6.2 and 6.3 until the aneurysm is filled as visualized by fluoroscopy. A separate liquid contrast agent can be used during the procedure as needed to determine extent of aneurysm fill.

During delivery, the catheter is preferably held in place in the aneurysm under conditions which minimize movement of the catheter.

In one embodiment, the embolizing composition preferably has a low viscosity of about 1 to 150 centistokes at 40°C.

In another embodiment, the embolizing composition preferably has a viscosity at 40°C of at least about 150 centistokes; more preferably from about 1,000 to about 20,000 centistokes; even more preferably from about 1,000 to 4,000 centistokes; still more preferably about 2,000 to 3,000 centistokes; and most preferably, about 2,500 centistokes. Particularly preferred viscosities at 40°C include 2,300 centistokes, 2,500 centistokes, and 3,200 centistolces. The viscosity is such that the biocompatible polymer precipitate forms a coherent mass (precipitate) at the distal tip of the catheter and does not form strings or similar structures susceptible to breakage. In a preferred embodiment, the viscosity is such that the embolizing material forms a dense spheroidal solid mass within the aneurysm or other vascular site without requiring the use of a flow-arresting device.

When introduced into the vascular site, the biocompatible solvent diffuses rapidly into the blood and a solid precipitate forms which precipitate is the water-insoluble polymer

### Utility

The compositions described herein are useful in embolizing mammalian blood vessels which, in turn, can be used to prevent/control bleeding (*e.g*., organ bleeding, gastrointestinal bleeding, vascular bleeding, bleeding associated with an aneurysm) or to ablate diseased tissue (*e.g*., tumors, AVMs, etc.). Accordingly, these compositions find use in human and other mammalian subjects requiring embolization of blood vessels.

It is contemplated that these compositions can be employed as a carrier for a compatible pharmaceutically-active compound wherein this compound is delivered *in vivo* for subsequent release. Such compounds include, by way of example only, antibiotics, antiinflammatory agents, chemotherapeutic agents, growth factors, and the like.

The following examples are set forth to illustrate the claimed invention and are not to be construed as a limitation thereof.

### EXAMPLES

Unless otherwise stated, all temperatures are in degrees Celsius. Also, in these examples and elsewhere, the following abbreviations have the following meanings:
- cc =: cubic centimeter
- cm =: centimeter
- cSt =: centistoke
- DMSO =: dimethylsulfoxide
- EVOH =: Ethylene vinyl alcohol copolymer
- g =: gm
- ID =: Internal diameter
- in.: = inch
- mg =: milligram
- min. =: minute
- mL =: milliliter
- mm =: millimeter
- OD =: outer diameter
- psi =: pounds per square inch
- sec. =: second
- µm =: micron

### EXAMPLE 1

The purpose of this example is to demonstrate the preparation of the embolic compositions discussed in the latter examples.

### Procedure

1. To a vial, 0.06 g EVOH was added to 1.1 g DMSO and 0.33 g tantalum. The composition was stirred as necessary to dissolve the EVOH. The resulting composition was labeled "Sample #1."
2. Each subsequent composition was prepared as was Sample #1, but with an increase in tantalum of 0.05 g over the previous Sample. The resulting compositions were labeled accordingly.

### Results

| Sample # | EVOH (g) | DMSO (g) | Tantalum (g) | % EVOH | % DMSO | % Tantalum | Ratio EVOH/tantalum |
|---|---|---|---|---|---|---|---|
| 1 | .06 | 1.1 | .33 | 4.02 | .8 | 22.1 | .182 |
| 2 | .06 | 1.1 | .38 | 3.90 | 71.4 | 24.7 | .158 |
| 3 | .06 | 1.1 | .43 | 3.78 | 69.2 | 27.0 | .140 |
| 4 | .06 | 1.1 | .48 | 3.66 | 67.0 | 29.3 | .125 |
| 5 | .06 | 1.1 | .53 | 3.55 | 65.1 | 31.4 | .113 |
| 6 | .06 | 1.1 | .58 | 3.45 | 63.2 | 33.3 | .103 |
| 7 | .06 | 1.1 | .63 | 3.35 | 61.5 | 35.2 | .095 |
| 8 | .06 | 1.1 | .68 | 3.26 | 59.8 | 37.0 | .088 |
| 9 | .06 | 1.1 | .73 | 3.17 | 58.2 | 38.6 | .082 |
| 10 | .06 | 1.1 | .78 | 3.09 | 56.7 | 40.2 | .077 |
| 11 | .06 | 1.1 | .83 | 3.02 | 55.3 | 41.7 | .072 |
| 12 | .06 | 1.1 | .88 | 2.94 | 53.9 | 43.1 | .068 |
| 13 | .06 | 1.1 | .93 | 2.87 | 52.6 | 44.5 | .065 |
| 14 | .06 | 1.1 | .98 | 2.80 | 51.4 | 45.8 | .061 |
| 15 | .06 | 1.1 | 1.03 | 2.74 | 50.2 | 47.0 | .058 |
| 16 | .06 | 1.1 | 1.08 | 2.68 | 49.1 | 48.2 | .056 |
| 17 | .06 | 1.1 | 1.13 | 2.62 | 48.0 | 49.3 | .053 |
| 18 | .06 | 1.1 | 1.18 | 2.56 | 47.0 | 50.4 | .051 |
| 19 | .06 | 1.1 | 1.23 | 2.51 | 46.0 | 51.5 | .049 |
| 20 | .06 | 1.1 | 1.28 | 2.46 | 45.1 | 52.5 | .047 |
| 21 | .06 | 1.1 | 1.33 | 2.41 | 44.2 | 53.4 | .045 |

### EXAMPLE 2

The purpose of this example is to quantitatively assess the difference in visibility under fluoroscopy (*i.e*., radiopacity) between: conventional embolic compositions, comprising no more than 40% contrast agent (typified by Sample #1); compositions comprising a higher level of contrast agent such that the ratio of polymer to contrast agent is equal to or greater than 0.055 (exemplified by Sample #10); and a wire.

The radiopacity of each material was analyzed using fluoroscopy. Radiopacity was determined based on the pixel density generated (the darker the image, the lower the pixel count), as seen by the fluoroscope's detector. By comparing the pixel counts of Sample #1 and Sample #10, viewed side-by-side, in combination with the standard (a metal wire), a relative assessment of expected fluoroscopic radiopacity was determined.

### Procedure

1. Prepare Samples #1 and #10, in vials.
2. Mix all Samples for at least 20 minutes.
3. Aspirate Samples into 1 mL syringe. Cut 0.025" ID silicone tubing into 3" lengths.
4. Inject Samples into tubing, completely filling lumen.
5. Immediately transfer Samples to the fluoroscope.
6. Visualize Samples with fluoroscope and record results.

### Results

| Sample # | Peak Pixel Count | Absolute % Difference | Relative % Difference |
|---|---|---|---|
| 1 | 136 | 0.7 | 0.8 |
| 10 | 82 | 40.1 | 46.6 |

Figure 1 demonstrates that compositions comprising a higher level of contrast agent such that the ratio of polymer to contrast agent is equal to or greater than 0.055 have significantly better radiopacity than do conventional embolic compositions.

### EXAMPLE 3

The purpose of this example is to ascertain whether compositions comprising higher levels of contrast agent such that the ratio of polymer to contrast agent is equal to or greater than 0.055 can be encapsulated into the polymer precipitate such that this precipitate is cohesive.

The cohesiveness of the precipitate is measured by determining the amount of particulate shedding in accordance with U.S. Pharmacopeia standard "USP XXV <788> Particulate Matter In Injections, Large Volume Parenterals (LVP)." This standard is used to enumerate subvisible extraneous particles within specific size ranges. Specifically, this standard measures whether more than 25 particles per mL of test solution measure greater than or equal to 10 µm and whether more than 3 particles per mL of test solution measure greater than or equal to 25 µm. This standard is applied to injectable solutions, which should be essentially free from particles that can be observed on visual inspection.

### Procedure

The NaCl control and Samples #1, #10, #11, #13, and #15 were prepared. All six compositions were tested in an electronic, liquid-borne particle counting system that uses a light-obscuration sensor within a suitable sample-feeding device. Specifically, particle counts were obtained for the compositions with the instrument set to count in the cumulative (total) mode. The samples were mixed by inverting 25 times within 10 seconds, and degassed by sonication (at 80 to 120 watts) for 30 seconds, or by allowing to stand. The samples were then gently stirred, taking care not to introduce air bubbles or contamination. While continuing to stir, 3 consecutive volumes of not less than 5 mL each were withdrawn and particle counts obtained. Data from the first portion were discarded, and the data from the second two portions were averaged. Using the formula: (Pₛ - P_{b}) ÷ V₁ the number of particles in each mL was determined, where Pₛ is the average particle count obtained from the composition; P_{b} is the average particle count obtained from the control; and V₁ is the average volume (in mL) of the 4 portions tested.

### Results

| Sample # | Particle ≥ 10µ per 1 mL solution (Minus control) | Particle ≥ 25µ per 1 mL solution (Minus control) |
|---|---|---|
| 1 | 2 | 0 |
| 10 | 18 | 3 |
| 11 | 22 | 2 |
| 13 | 23 | 3 |
| 15 | 17 | 3 |

The foregoing results indicate that compositions comprising a higher level of contrast agent and having a ratio of polymer to contrast agent of 0.055 or greater form cohesive polymer precipitates, indicating that such compositions axe suitable for parenteral delivery.

### EXAMPLE 4

The purpose of this example is to determine whether compositions of this invention are suitable for controlled delivery via catheter.

Embolization of blood vessels is preferably accomplished via catheter techniques which permit the selective placement of the catheter at the vascular site to be embolized. In the case of aneurysms, the amount of embolic composition delivered should substantially fill, but not overfill, the aneurysmal sac. If less than the desired amount of composition is delivered, the patient is left with an active aneurysm, which can be more dangerous than the initial, untreated aneurysm. If more than the desired amount of composition is delivered, the composition may overflow into an adjoining blood vessel, and may possibly embolize an undesired location. Accordingly, the ability of the treating physician to control the catheter injection of the embolic composition is paramount to successful treatment.

### Procedure

Samples #1, #10, #12, #14, and #21 were prepared in vials. The vials were heated for 20 minutes. Each Sample was aspirated into a 1 cc syringe, which was attached directly to a pressure transducer block. The 1.5 F French microcatheter hub was attached directly to the transducer (not via the interface needle). The samples were injected through the microcatheter until they exited the distal tip. The injection was then continued at a rate of 0.1 mL/min. for an additional total injection volume of 0.35 mL. The peak pressure was recorded at 0.18 mL (half way through the injection cycle). After a two-minute wait time, the injection was resumed at a new rate of 0.3 mL/min. Similarly, the peak injection pressure was recorded at 0.18 mL. The recorded pressures at each injection rate were averaged.

### Results

| Sample # | Sample Size (n) | Pressure at 0.1 mL/min | Pressure at 0.3 mL/min |
|---|---|---|---|
| 1 | 5 | 13.4 | 43.1 |
| 10 | 5 | 18.2 | 51.1 |
| 12 | 1 | 17.2 | 52.5 |
| 14 | 1 | 19.1 | 59.0 |
| 21 | 1 | 21.4 | 62.0 |

The data obtained show a positive relation between increased contrast agent concentrations and increased injection pressures at the infusion rates tested. However, even the maximum injection pressure of 62.0 psi at the rapid infusion rate of 0.3 mL/min is well below the pressure capability of conventional catheters. Therefore, the small increase in injection pressure is not considered significant. It was also observed that viscosity did not change significantly.

Therefore, compositions of this invention can be injected smoothly, without blockage of the catheter, and are suitable for controlled delivery via catheter.

### EXAMPLE 5

The purpose of this example is to determine whether compositions of this invention solidify quickly enough to permit their use as embolic compositions.

As explained above, embolization of blood vessels is preferably accomplished via catheter techniques which permit the selective placement of the catheter at the vascular site to be embolized. The goal of such embolization is to deliver precisely the amount needed to fill the aneurysmal sac because, *inter alia,* overfilling the sac could result in unintended embolization of adjacent blood vessels.

During the embolization procedure, the use of a blood flow-arresting device (such as a balloon) is often employed. However, if such a flow-arresting device is used for too long, ischemia may result, as the tissue has been deprived of blood flow. Therefore, one must determine whether a potential embolic composition solidifies quickly enough so that the risk of ischemia will be minimized. In addition, when treating certain conditions (*e.g*., AVMs) it is desirable to deliver the embolic composition to the nidus only, not allowing the embolic composition to extend further into the vascular framework. Rapid solidification of the embolic composition facilitates such precise and contained delivery.

### Procedure

Samples #1 and #10 were prepared. Each Sample was aspirated into a 1 cc syringe. With the tip of the syringe aimed a few inches above a 150 cc glass beaker of saline, the Sample was slowly ejected out so that a droplet formed on the tip of the syringe. As more of the Sample was ejected out, the droplet grew bigger. Once a 3.0 mm droplet was formed, it was dropped into the saline bath and timed as it hit the saline. After 30 seconds, the droplet was removed from the saline with tweezers. The droplet was placed on a microscopic glass slide and immediately covered with another glass slide. Slowly and gently, pressure was applied to the top slide at both ends. As the droplet was flattened, it was observed whether a liquid composition appeared. Observation of a liquid composition indicated that the droplet had not fully solidified. This procedure was repeated for 1, 1.5, 2, 2.5, and 3 minutes.

### Results

| Time Interval (min) | Sample #1 | Sample #10 |
|---|---|---|
| 0.5 | 0/3 Solidified | 0/3 Solidified |
| 1.0 | 0/3 Solidified | 0/3 Solidified |
| 1.5 | 0/3 Solidified | 0/3 Solidified |
| 2.0 | 0/3 Solidified | 2/3 Solidified |
| 2.5 | 1/3 Solidified | 6/6 Solidified |
| 3.0 | 6/6 Solidified | N/A |

The data obtained indicate that the compositions of this invention are well suited to treat vascular conditions, including AVMs, because they solidify in a manner similar to conventional embolic compositions which are known to successfully treat such vascular conditions.

From the foregoing description, various modifications and changes in the composition and method will occur to those skilled in the art. All such modifications coming within the scope of the appended claims are intended to be included therein.

## Claims

1. A composition having a high amount of contrast agent, comprising :
a) a biocompatible polymer;
b) a biocompatible solvent; and
c) from 45 to 60 weight percent of a water-insoluble, biocompatible contrast agent;
wherein the ratio of biocompatible polymer to the water-insoluble biocompatible contrast agent is 0.055 or greater; and
further wherein the weight percent of each component is based on the total weight of the composition.

2. The composition according to Claim 1, wherein the said ratio of biocompatible polymer to the water-insoluble biocompatible contrast agent is 0.058 or greater.

3. The composition according to Claim 1, wherein the said ratio of biocompatible polymer to the water-insoluble biocompatible contrast agent is 0.070 or greater.

4. The composition according to Claim 1, wherein the water-insoluble biocompatible contrast agent is employed at a concentration of from greater than 45 to 55 weight percent, based on the total weight of the composition.

5. The composition according to Claim 1, wherein the average particle size of the water-insoluble biocompatible contrast agent is less than 5 microns.

6. The composition according to Claim 5, wherein the average particle size of the water-insoluble biocompatible contrast agent is from 2 microns to 3 microns.

7. The composition according to Claim 1, wherein the water-insoluble, biocompatible contrast agent is selected from the group consisting of barium sulfate, tantalum, tantalum oxide, gold, platinum and tungsten.

8. The composition according to Claim 1, wherein the biocompatible polymer is employed at a concentration of from 2 to 40 weight percent, based on the total weight of the composition.

9. The composition according to Claim 8, wherein the biocompatible polymer is employed at a concentration of from 2 to 30 weight percent, based on the total weight of the composition.

10. The composition according to Claim 9, wherein the biocompatible polymer is employed at a concentration of from 2 to 20 weight percent, based on the total weight of the composition.

11. The composition according to Claim 1, wherein the biocompatible polymer is selected from the group consisting of cellulose acetates, ethylene vinyl alcohol copolymers, hydrogels, polyacrylonitrile, polyvinylacetate, cellulose acetate butyrate, nitrocellulose, copolymers of urethane/carbonate, copolymers of styrene/maleic acid, and mixtures thereof.

12. The composition according to Claim 1, wherein the concentration of biocompatible solvent is from 20 weight percent to less than 58 weight percent, based on the total weight of the composition.

13. The composition according to Claim 12, wherein the concentration of biocompatible solvent is from 20 to 57 weight percent, based on the total weight of the composition.

14. The composition according to Claim 13, wherein the concentration of biocompatible solvent is from 40 to 55 weight percent, based on the total weight of the composition.

15. The composition according to Claim 1, wherein the biocompatible solvent is selected from the group consisting of dimethylsulfoxide ("DMSO"), ethanol, ethyl lactate, and acetone.

16. A kit of parts comprising :
a) an embolic composition which comprises
i) a biocompatible polymer ;
ii) a biocompatible solvent; and
iii) from 45 to 60 weight percent of a water- insoluble, biocompatible contrast agent ;
wherein the ratio ofbiocompatible polymer to the water-insoluble biocompatible contrast agent is 0.055 or greater,
and further wherein the weight percent of each component is based on the total weight of the composition; and
b) a catheter.

17. The kit of parts according to Claim 16, which further comprises a microballoon catheter to attenuate or arrest blood flow.

## Patentansprüche

1. Zusammensetzung, umfassend eine große Menge von Kontrastmittel, enthaltend:
a) ein biokompatibles Polymer;
b) ein biokompatibles Lösungsmittel; und
c) von 45 bis 60 Gew.-% eines wasserunlöslichen, biokompatiblen Kontrastmittels;
wobei das Verhältnis des biokompatiblen Polymers zum wasserunlöslichen biokompatiblen Kontrastmittel mindestens 0.055 beträgt; und
wobei das Gew.-% jeder Komponente auf das Gesamtgewicht der Zusammensetzung bezogen ist.

2. Zusammensetzung gemäß Anspruch 1, wobei das Verhältnis des biokompatiblen Polymers zum wasserunlöslichen, biokompatiblen Kontrastmittel mindestens 0.058 beträgt.

3. Zusammensetzung gemäß Anspruch 1, wobei das Verhältnis des biokompatiblen Polymers zum wasserunlöslichen, biokompatiblen Kontrastmittel mindestens 0.070 beträgt.

4. Zusammensetzung gemäß Anspruch 1, wobei das wasserunlösliche, biokompatible Kontrastmittel in einer Konzentration von mehr als 45 bis 55 Gew.-% zur Anwendung kommt, bezogen auf das Gesamtgewicht der Zusammensetzung.

5. Zusammensetzung gemäß Anspruch 1, wobei das wasserunlösliche, biokompatible Kontrastmittel eine durchschnittliche Teilchengröße von weniger als 5 Mikron (µm) ausweist.

6. Zusammensetzung gemäß Anspruch 5, wobei das wasserunlösliche, biokompatible Kontrastmittel eine durchschnittliche Teilchengröße von 2 bis 3 Mikrons (µm) ausweist.

7. Zusammensetzung gemäß Anspruch 1, wobei das wasserunlösliche, biokompatible Kontrastmittel ausgewählt aus der Gruppe ist, die aus Bariumsulfat, Tantal, Tantaloxid, Gold, Platin und Wolfram besteht.

8. Zusammensetzung gemäß Anspruch 1, wobei das biokompatible Polymer in einer Konzentration von 2 bis 40 Gew.-% zur Anwendung kommt, bezogen auf das Gesamtgewicht der Zusammensetzung.

9. Zusammensetzung gemäß Anspruch 8, wobei das biokompatible Polymer in einer Konzentration von 2 bis 30 Gew.-% zur Anwendung kommt, bezogen auf das Gesamtgewicht der Zusammensetzung.

10. Zusammensetzung gemäß Anspruch 9, wobei das biokompatible Polymer in einer Konzentration von 2 bis 20 Gew.-% zur Anwendung kommt, bezogen auf das Gesamtgewicht der Zusammensetzung.

11. Zusammensetzung gemäß Anspruch 1, wobei das biokompatible Polymer ausgewählt aus der Gruppe ist, die aus Celluloseacetat, Copolymeren von Ethylenvinylalkohol, Hydrogelen, Polyacrylnitril, Polyvinylacetat, Celluloseacetatbutyrat, Nitrocellulose, Urethan/Carbonat- Copolymeren, Styrol/Maleinsäure-Copolymeren und Mischungen davon besteht.

12. Zusammensetzung gemäß Anspruch 1, wobei das biokompatible Lösungsmittel in einer Konzentration von 20 bis weniger als 58 Gew.-% zur Anwendung kommt, bezogen auf das Gesamtgewicht der Zusammensetzung.

13. Zusammensetzung gemäß Anspruch 12, wobei das biokompatible Lösungsmittel in einer Konzentration von 20 bis 57 Gew.-% zur Anwendung kommt, bezogen auf das Gesamtgewicht der Zusammensetzung.

14. Zusammensetzung gemäß Anspruch 13, wobei das biokompatible Lösungsmittel in einer Konzentration von 40 bis 55 Gew.-% zur Anwendung kommt, bezogen auf das Gesamtgewicht der Zusammensetzung.

15. Zusammensetzung gemäß Anspruch 1, wobei das biokompatible Lösungsmittel ausgewählt aus der Gruppe ist, die aus Dimethylsulfoxid ("DMSO"), Ethanol, Ethyllactat und Aceton besteht.

16. Kit von Teilen enthaltend:
a) eine Embolisierungs-Zusammensetzung, die umfasst
i. ein biokompatibles Polymer;
ii. ein biokompatibles Lösungsmittel; und
iii. von 45 bis 60 Gew.-% eines wasserunlöslichen, biokompatiblen Kontrastmittels;
wobei das Verhältnis des biokompatiblen Polymers zum wasserunlöslichen biokompatiblen Kontrastmittel mindestens 0.055 beträgt; und
wobei das Gew.-% jeder Komponente auf das Gesamtgewicht der Zusammensetzung bezogen ist, und
b) ein Katheter.

17. Kit von Teilen gemäß Anspruch 16, welches ferner einen Mikroballonkatheter umfasst, der dafür geeignet ist, den Blutstrom abzuschwächen oder zu unterbrechen.

## Revendications

1. Composition ayant une quantité élevée d'agent de contraste, comprenant :
a) un polymère biocompatible ;
b) un solvant biocompatible ; et
c) de 45 à 60 pour cent en poids d'un agent de contraste biocompatible insoluble dans l'eau ;
dans laquelle le rapport du polymère biocompatible sur l'agent de contraste biocompatible insoluble dans l'eau est 0,055 ou plus ; et
en outre dans laquelle le pourcentage en poids de chaque composant est basé sur le poids total de la composition.

2. Composition selon la revendication 1, dans laquelle ledit rapport du polymère biocompatible sur l'agent de contraste biocompatible insoluble dans l'eau est 0,058 ou plus.

3. Composition selon la revendication 1, dans laquelle ledit rapport du polymère biocompatible sur l'agent de contraste biocompatible insoluble dans l'eau est 0,070 ou plus.

4. Composition selon la revendication 1, dans laquelle l'agent de contraste biocompatible insoluble dans l'eau est employé à une concentration de plus de 45 à 55 pour cent en poids, sur la base du poids total de la composition.

5. Composition selon la revendication 1, dans laquelle la taille moyenne de particules de l'agent de contraste biocompatible insoluble dans l'eau est inférieure à 5 micromètres.

6. Composition selon la revendication 5, dans laquelle la taille moyenne de particules de l'agent de contraste biocompatible insoluble dans l'eau est de 2 micromètres à 3 micromètres.

7. Composition selon la revendication 1, dans laquelle l'agent de contraste biocompatible insoluble dans l'eau est choisi parmi le groupe consistant en le sulfate de baryum, le tantale, l'oxyde de tantale, l'or, le platine et le tungstène.

8. Composition selon la revendication 1, dans laquelle le polymère biocompatible est employé à une concentration de 2 à 40 pour cent en poids, sur la base du poids total de la composition.

9. Composition selon la revendication 8, dans laquelle le polymère biocompatible est employé à une concentration de 2 à 30 pour cent en poids, sur la base du poids total de la composition.

10. Composition selon la revendication 9, dans laquelle le polymère biocompatible est employé à une concentration de 2 à 20 pour cent en poids, sur la base du poids total de la composition.

11. Composition selon la revendication 1, dans laquelle le polymère biocompatible est choisi parmi le groupe consistant en des acétates de cellulose, des copolymères d'éthylène-alcool vinylique, des hydrogels, un polyacrylonitrile, un poly(acétate de vinyle), un acétobutyrate de cellulose, la nitrocellulose, des copolymères d'uréthane/carbonate, des copolymères de styrène/acide maléique, et des mélanges de ceux-ci.

12. Composition selon la revendication 1, dans laquelle la concentration du solvant biocompatible est de 20 pour cent en poids à moins de 58 pour cent en poids, sur la base du poids total de la composition.

13. Composition selon la revendication 12, dans laquelle la concentration du solvant biocompatible est de 20 à 57 pour cent en poids, sur la base du poids total de la composition.

14. Composition selon la revendication 13, dans laquelle la concentration du solvant biocompatible est de 40 à 55 pour cent en poids, sur la base du poids total de la composition.

15. Composition selon la revendication 1, dans laquelle le solvant biocompatible est choisi parmi le groupe consistant en le diméthylsulfoxyde (« DMSO »), l'éthanol, le lactate d'éthyle et l'acétone.

16. Kit de pièces comprenant :
a) une composition embolique qui comprend
i) un polymère biocompatible ;
ii) un solvant biocompatible ; et
iii) de 45 à 60 pour cent en poids d'un agent de contraste biocompatible insoluble dans l'eau ;
dans laquelle le rapport du polymère biocompatible sur l'agent de contraste biocompatible insoluble dans l'eau est 0,055 ou plus ;
et en outre dans laquelle le pourcentage en poids de chaque composant est basé sur le poids total de la composition ; et
b) un cathéter.

17. Kit de pièces selon la revendication 16, qui comprend en outre un cathéter à microballon pour atténuer ou arrêter un flux sanguin.
